# FASCICULE DE BREVET EUROPEEN

(11) **EP 3 801 213 B1**
(45) Date de publication et mention de la délivrance du brevet: **22.06.2022**
(21) Numéro de dépôt: 19739677.3
(22) Date de dépôt: 28.05.2019
(51) Int. Cl.: A61B 5/11, G16H 40/63, A61B 5/145, A61B 5/00

(54) **PROCEDE POUR DETECTER UNE QUANTITE DE NO PRODUITE PAR LE SUJET ETUDIE ET APPAREIL POUR LA MISE EN OEUVRE DUDIT PROCEDE**
VERFAHREN ZUR ERFASSUNG EINER VON EINER TESTPERSON ERZEUGTEN MENGE AN NO UND VORRICHTUNG ZUR DURCHFÜHRUNG DES VERFAHRENS
METHOD FOR DETECTING A QUANTITY OF NO PRODUCED BY THE SUBJECT UNDER TEST, AND APPARATUS FOR CARRYING OUT SAID METHOD

(30) Priorité: 28.05.2018 FR 1854495
(43) Date de publication de la demande: 14.04.2021
(73) Titulaire: NOPTRACK, 81860 Castres (FR)
(72) Inventeur: RIVIERE, Philippe, 31500 Toulouse (FR); VIALARD, Luc, 31400 Toulouse (FR); PEREZ, Yoann, 65410 Sarrancolin (FR); DAUMAS, Frédéric, 31120 Pinsaguel (FR); AUBAGNAC, Jean-Christophe, 31420 Saint-Jean (FR); LABRUNEE, Marc, 31400 Toulouse (FR); FAVRE, Gilles, 31270 Cugnaux (FR); AMATORE, Christian, 75013 PARIS (FR)
(74) Mandataire: Loyer & Abello
(86) Numéro de dépôt international: PCT/FR2019/051261
(87) Numéro de publication internationale: WO 2019/229380

(56) Documents cités:
- WO-A2-98/08480
- WO-A2-2016/061362
- JUNGIL CHOI ET AL: "Skin-interfaced systems for sweat collection and analytics", SCIENCE, vol. 4, no. 2, 16 février 2018 (2018-02-16), page eaar3921, XP055526287, ISSN: 0036-8075, DOI: 10.1126/sciadv.aar3921
- YEJIN HA ET AL: "Measurements of Location-Dependent Nitric Oxide Levels on Skin Surface in relation to Acupuncture Point", EVIDENCE-BASED COMPLEMENTARY AND ALTERNATIVE MEDICINE, vol. 2012, 1 janvier 2012 (2012-01-01), pages 1-7, XP055524807, ISSN: 1741-427X, DOI: 10.1155/2012/781460
- ALBERT MAAREK ET AL: "Detection of neuropathy using a sudomotor test in type 2 diabetes", DEGENERATIVE NEUROLOGICAL AND NEUROMUSCULAR DISEASE, 9 janvier 2015 (2015-01-09), page 1, XP055525495, ISSN: 1179-9900, DOI: 10.2147/DNND.S75857
- BYEONG WAN AN ET AL: "Smart Sensor Systems for Wearable Electronic Devices", POLYMERS, vol. 9, no. 12, 25 juillet 2017 (2017-07-25), page 303, XP055526164, DOI: 10.3390/polym9080303
- Youngmi Lee ET AL: "Improved Planar Amperometric Nitric Oxide Sensor Based on Platinized Platinum Anode. 1. Experimental Results and Theory When Applied for Monitoring NO Release from Diazeniumdiolate-Doped Polymeric Films - Analytical Chemistry (ACS Publications)", , 1 janvier 2004 (2004-01-01), XP055526149, Extrait de l'Internet: URL:https://pubs.acs.org/doi/10.1021/ac035 064h [extrait le 2018-11-22]
- BRUCH-GERHARZ D ET AL: "Nitric oxide in human skin: current status and future prospects", JOURNAL OF INVESTIGATIVE DERMATOLOGY, ELSEVIER, NL, vol. 110, no. 1, 1 janvier 1998 (1998-01-01), pages 1-7, XP002254968, ISSN: 0022-202X, DOI: 10.1046/J.1523-1747.1998.00084.X
- MA ET AL: "Evidence of enhanced non-enzymatic generation of nitric oxide on the skin surface of acupuncture points: An innovative approach in humans", NITRIC OXIDE: BIOLOGY AND CHEMISTRY, ACADEMIC PRESS, AMSTERDAM, NL, vol. 17, no. 2, 18 août 2007 (2007-08-18), pages 60-68, XP022207173, ISSN: 1089-8603, DOI: 10.1016/J.NIOX.2007.05.004
- MALLIKA BARIYA ET AL: "Wearable sweat sensors", NATURE ELECTRONICS, vol. 1, no. 3, 1 mars 2018 (2018-03-01), pages 160-171, XP055525597, ISSN: 2520-1131, DOI: 10.1038/s41928-018-0043-y
- JOHNE LEWIS ET AL: "New method of sudomotor function measurement to detect microvascular disease and sweat gland nerve or unmyelinated C fiber dysfunction in adults with retinopathy", JOURNAL OF DIABETES & METABOLIC DISORDERS, BIOMED CENTRAL LTD, LONDON, UK, vol. 16, no. 1, 12 juin 2017 (2017-06-12), pages 1-10, XP021245874, DOI: 10.1186/S40200-017-0307-5
- YEJIN HA ET AL: "Insertable NO/CO Microsensors Recording Gaseous Vasomodulators Reflecting Differential Neuronal Activation Level with Respect to Seizure Focus", ACS CHEMICAL NEUROSCIENCE, vol. 8, no. 9, 5 juillet 2017 (2017-07-05) , pages 1853-1858, XP055524923, US ISSN: 1948-7193, DOI: 10.1021/acschemneuro.7b00141
- SALZITSA ANASTASOVA ET AL: "A wearable multisensing patch for continuous sweat monitoring", BIOSENSORS AND BIOELECTRONICS, vol. 93, 1 juillet 2017 (2017-07-01), pages 139-145, XP055482880, AMSTERDAM, NL ISSN: 0956-5663, DOI: 10.1016/j.bios.2016.09.038
- RICHARD WELLER ET AL: "Nitric Oxide Is Generated on the Skin Surface by Reduction of Sweat Nitrate", JOURNAL OF INVESTIGATIVE DERMATOLOGY, vol. 107, no. 3, 1 septembre 1996 (1996-09-01), pages 327-331, XP055205544, DOI: 10.1111/1523-1747.ep12363167
- Kichang Lee ET AL: "Role of nitric oxide in methacholine-induced sweating and vasodilation in human skin", Journal of Applied Physiology, 1 avril 2006 (2006-04-01), pages 1355-1360, XP055525486, United States DOI: 10.1152/japplphysiol.00122.2005 Extrait de l'Internet: URL:https://pdfs.semanticscholar.org/c834/ 1f4a660265a12e6e0bf87ba563ab9b9fe1ce.pdf
- VINCENZO F. CURTO ET AL: "Real-time sweat pH monitoring based on a wearable chemical barcode micro-fluidic platform incorporating ionic liquids", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, vol. 171-172, 1 août 2012 (2012-08-01), pages 1327-1334, XP055451404, NL ISSN: 0925-4005, DOI: 10.1016/j.snb.2012.06.048
- HNIN YIN YIN NYEIN ET AL: "A Wearable Microfluidic Sensing Patch for Dynamic Sweat Secretion Analysis", ACS SENSORS, vol. 3, no. 5, 9 mai 2018 (2018-05-09), pages 944-952, XP055525587, ISSN: 2379-3694, DOI: 10.1021/acssensors.7b00961
- KRAMER RYAN M ET AL: "Analytical determination and detection of individual odor signatures", SENSING TECHNOLOGIES FOR GLOBAL HEALTH, MILITARY MEDICINE, DISASTER RESPONSE, AND ENVIRONMENTAL MONITORING II; AND BIOMETRIC TECHNOLOGY FOR HUMAN IDENTIFICATION IX, SPIE, 1000 20TH ST. BELLINGHAM WA 98225-6705 USA, vol. 8371, no. 1, 11 mai 2012 (2012-05-11) , pages 1-14, XP060002731, DOI: 10.1117/12.919921
- YEJIN HA ET AL: "Amperometric Microsensors Monitoring Glutamate-Evoked In Situ Responses of Nitric Oxide and Carbon Monoxide from Live Human Neuroblastoma Cells", SENSORS, vol. 17, no. 7, 19 juillet 2017 (2017-07-19), page 1661, XP055524813, DOI: 10.3390/s17071661
- GF CLOUGH ET AL: "Measurement of nitric oxide concentration in human skin in vivo using dermal microdialysis", EXPERIMENTAL PHYSIOLOGY, vol. 83, no. 3, 1 mai 1998 (1998-05-01), pages 431-434, XP055526121, GB ISSN: 0958-0670, DOI: 10.1113/expphysiol.1998.sp004126
- LUO XIAOJIN ET AL: "A Wearable Amperometric Biosensor on a Cotton Fabric for Lactate", IEEE ELECTRON DEVICE LETTERS, IEEE SERVICE CENTER, NEW YORK, NY, US, vol. 39, no. 1, 1 janvier 2018 (2018-01-01), pages 123-126, XP011675011, ISSN: 0741-3106, DOI: 10.1109/LED.2017.2777474 [extrait le 2017-12-26]

## Description

L'invention concerne un procédé et un appareil pour détecter, sur un sujet humain ou animal ou végétal, mort ou vivant, par exemple un état physiologique et/ou un état physiopathologique du sujet. L'invention concerne également un appareil autonome permettant de mesurer le NO dans le but de déterminer un état physiologique ou physiopathologique du sujet comme par exemple diagnostiquer et/ou prévenir l'apparition de pathologies liées à cette molécule et/ou suivre l'efficacité thérapeutique.

On sait que le monoxyde d'azote est un gaz qui constitue un messager intercellulaire. Le NO joue un rôle important dans la protection contre l'apparition et la progression de certaines maladies cardio-vasculaires, de certaines maladies neurodégénératives, l'hypertension artérielle pulmonaire, ou encore l'oncogenèse. Les pathologies cardiovasculaires associées incluent l'hypercholestérolémie, l'hypertension et le diabète. La maladie sous-jacente pour la plupart des maladies cardio-vasculaires (vaisseaux cérébraux, artères coronaires, ischémie des membres inférieurs) est un système endothélial dysfonctionnel, qui est associé à l'artériosclérose pouvant conduire à des pathologies thrombotiques et ischémiques. YEJIN HA ET AL: EVIDENCE-BASED COMPLEMENTARY AND ALTERNATIVE MEDICINE, 1 janvier 2012 (2012-01-01) divulgue des méthodes et appareils mesurant le NO aux points des acupunctures

Le rôle cardio-protectif du NO inclut notamment la régulation de la tension et le tonus vasculaire, l'inhibition d'accumulation plaquettaire, l'adhérence leucocytaire et la prolifération de cellules des fibres musculaires lisses. Le NO est aussi impliqué dans l'inflammation bronchique ; on a notamment mesuré que la concentration de NO est plus importante dans l'air expiré chez les sujets asthmatiques que chez les sujets non asthmatiques. On a aussi constaté que le NO est impliqué, en fonction de sa concentration, dans l'apparition ou la régression de tumeurs. On a aussi constaté que le NO est impliqué dans la pathologie de la maladie d'Alzheimer. L'ensemble des maladies concernées par le NO s'inscrit dans les affections longue durée, dont le coût annuel devient plus important chaque année et rend nécessaire des outils de prévention et de prédiction d'apparition de ces maladies.

En physiologie, le monoxyde d'azote est un très bon indicateur de la croissance et/ou de la souffrance musculaire et donc du suivi de l'entrainement physique des sportifs ainsi que de toute personne qui se soumet à une activité physique. Ainsi en mesurant la production de monoxyde d'azote, on peut éviter les blessures dues au surentrainement et/ou favoriser la prise de NO pour favoriser la croissance musculaire et augmenter les performances sportives. Cela s'applique aussi bien à l'homme qu'aux animaux.

Dans le cas d'une maladie cardio-vasculaire, les dispositifs actuellement existants et les outils de prévention et de prédiction sont soit limités à une mesure indirecte du NO du patient au repos, soit limités à une mesure directe différée de plusieurs heures par rapport à une observation d'un problème pathologique. Dans tous les cas, les mesures ne peuvent être réalisées que dans un environnement médicalisé.

Selon la présente invention, on propose un appareil permettant une mesure directe, continue et immédiate du NO dans un liquide biologique, tel que la sueur, au niveau d'un épiderme, tel que la peau, sur un sujet tel qu'un patient ou un mammifère, dans sa vie de tous les jours ou au lors d'une prescription médicale en milieu médical, éventuellement sur plusieurs jours et en toutes conditions environnementales, notamment en matière de pression, d'humidité et de température. Un tel appareil permet de détecter et de déduire l'évolution d'un état physiologique ou pathophysiologique comme un risque d'apparition de pathologies ou le suivi thérapeutique.

La présente invention a pour objet un procédé pour détecter, sur un sujet, notamment humain ou animal ou végétal, le sujet étant mort ou vivant, une quantité de NO produite par ledit sujet au cours d'une séquence d'un état d'activité prédéfini, caractérisé en ce qu'on choisit une zone d'investigation d'un épiderme dudit sujet, on y repère, de façon directe et continue, la production de NO dissous dans un liquide biologique provenant de l'épiderme au moyen d'un appareil formé d'une première partie, portée par ladite zone d'investigation et maintenue sur elle de façon étanche, cette première partie étant solidarisée d'un élément sensible, qui assure la détection du NO au moyen d'un capteur électrochimique, et que l'on envoie par ledit capteur électrochimique, grâce à un générateur d'énergie associé audit élément sensible, un signal, dont la lecture permet la détection souhaitée.

On entend par « NO dans un liquide biologique » que le NO est dissous dans un liquide biologique.

On entend par « épiderme » le tissu végétal superficiel formant une couche protectrice des parties aériennes d'un végétal ou la couche superficielle de la peau chez l'homme et les animaux.

On entend par « liquide biologique provenant de l'épiderme », tout liquide produit par le sujet et excrété via ou par l'épiderme du sujet. Ce liquide biologique est par exemple l'exsudat chez les végétaux ou la sueur chez l'homme et les animaux.

On entend par « de façon étanche » que les gaz, les liquides et les micro-organismes tels que bactéries ou virus, situés à l'extérieur de la zone d'investigation ne peuvent pas entrer dans la zone d'investigation. L'étanchéité du contact entre la première partie et la zone d'investigation assure que le NO détecté provient du liquide biologique produit par la zone d'investigation, et non d'un flux venant de l'extérieur.

On entend par « séquence » une séquence temporelle c'est-à-dire un intervalle de temps.

On entend par « état d'activité prédéfini » l'état dans lequel se trouve le sujet, par exemple faisant un exercice musculaire, endormi, assis, courant, immobile, voire mort, ...

Selon un mode de réalisation, le procédé permet de détecter au moins un paramètre associé à un état physiologique ou une pathologie.

Selon un mode de réalisation, la première partie comporte un corps fibreux pour amener le liquide biologique de la zone d'investigation à l'élément sensible grâce aux forces de capillarité.

Selon un mode de réalisation, la première partie comporte en outre un filtre configuré pour filtrer le liquide biologique à une entrée de l'élément sensible afin d'éviter de fausser la détection de NO par des éléments perturbateurs contenus dans le liquide biologique.

Selon un mode de réalisation, le filtre est une membrane de type eugénol.

Selon des modes de réalisation, le corps fibreux peut être, un matériau tissé, et un matériau non tissé tel que le coton.

Dans une variante de procédé, on met en œuvre au moins un capteur électrochimique, qui fournit un signal comme résultat d'une mesure électrochimique faite en utilisant le liquide biologique, notamment la sueur ou l'exsudat produit par le sujet dans la zone d'investigation, comme électrolyte entre deux électrodes de travail portées par un support planaire isolant.

Selon un mode de réalisation, le support planaire isolant comprend un matériau choisi parmi les élastomères tels que le polydiméthylsiloxane (PDMS), les polyimides, les résines époxydes et le parylène.

On peut prévoir que, dans le procédé selon une variante de l'invention, on associe aux deux électrodes de travail, une électrode de référence.

Selon un mode de réalisation, l'électrode de référence est une électrode au chlorure d'argent (AgCI).

On peut aussi prévoir que l'élément sensible comporte une pluralité de capteurs électrochimiques semblables, dont les signaux sont associés pour améliorer le signal de sortie.

On peut prévoir que le tracé des électrodes par rapport à leur support, suit une courbe de Hilbert, pour améliorer la puissance du signal de sortie par unité de surface du support.

Selon un mode de réalisation, le tracé des électrodes par rapport à leur support, peut suivre un autre type de courbe choisi parmi une courbe de Peano, une courbe de Sierpinski, une courbe de Moore et une courbe de Lebesque, aussi dans le but d'améliorer la force du signal de sortie par unité de surface du support. On peut prévoir que l'on effectue les mesures au droit d'orifices prévus dans le support planaire, qui se trouve au droit des tracés conducteurs des électrodes. Dans une réalisation intéressante, les électrodes sont constituées de dépôts métalliques, notamment en argent (Ag), en or (Au), en platine (Pt), en noir de platine, ou de dépôts de graphène dopés par des nanoparticules d'argent (Ag) ou d'or (Au), les nanoparticules étant fonctionnalisées par des liants du NO, notamment la guanylyl-cyclase ou des porphyrines.

Selon un mode de réalisation, les dépôts métalliques en or sont effectués en amas ou effectués en suivant un motif précis, par exemple un motif hexagonal.

Pour une mise en œuvre du procédé selon l'invention, on peut prévoir que l'appareil comporte en outre une deuxième partie disposée au-dessus de la première partie, la deuxième partie renfermant une électronique pour recueillir les mesures brutes du capteur électrochimique, les traduire en concentration de NO et assurer la transmission du signal avec éventuellement d'autres paramètres liés à l'environnement.

On peut prévoir que, dans le procédé selon l'invention, l'appareil effectue et transmet des mesures à une fréquence fonction de l'état d'activité du sujet, cet état étant repéré au moyen d'un module gyroscopique et/ou accélérométrique de la deuxième partie de l'appareil.

Selon un mode de réalisation, l'appareil comprend un module de géolocalisation.

L'invention concerne également un appareil de détection pour détecter, sur un sujet, une quantité de NO produite par ledit sujet au cours d'une séquence d'un état d'activité prédéfini, ledit appareil comportant une première partie destinée à être portée par une zone d'investigation d'un épiderme dudit sujet et maintenue sur elle de façon étanche pour repérer de façon directe et continue, la production de NO dans un liquide biologique provenant de l'épiderme, la première partie étant solidarisée d'un élément sensible, qui assure la détection de NO au moyen d'un capteur électrochimique, et une deuxième partie configurée pour envoyer, grâce à un générateur d'énergie associé audit élément sensible, un signal, dont la lecture permet la détection souhaitée.

On peut prévoir que l'élément sensible fournit le signal comme résultat d'une mesure électrochimique faite en utilisant le liquide biologique, produit par le sujet dans la zone d'investigation, comme électrolyte entre deux électrodes de travail portées par un support planaire isolant.

Dans une variante susmentionnée, on peut prévoir qu'une électrode de référence soit associée aux deux électrodes de travail.

Selon un mode de réalisation, le support planaire isolant comprend au moins un microcanal de sorte à guider le liquide biologique vers le capteur électrochimique. On peut prévoir que l'élément sensible comporte une pluralité de capteurs électrochimiques semblables, dont les signaux sont associés pour améliorer le signal de sortie.

Selon un mode de réalisation, l'élément sensible comporte une pluralité de capteurs électrochimiques réparties en une pluralité d'unités sensibles et en ce que chaque unité sensible est configurée pour détecter au moins une espèce chimique. L'élément sensible peut alors détecter plusieurs espèces chimiques différentes.

Selon un mode de réalisation, le support planaire isolant comprend une pluralité de microcanaux, et chaque canal comprend une unité sensible.

On peut prévoir que dans l'appareil selon l'invention, le tracé des électrodes par rapport à leur support, suit une courbe de Hilbert pour améliorer la force du signal de sortie par unité de surface du support.

Dans un tel appareil, les mesures sont effectuées au droit d'orifices prévus dans le support planaire, qui se trouve au droit des tracés conducteurs des électrodes. On peut prévoir que, dans l'appareil selon l'invention, les électrodes de travail sont constituées de dépôts métalliques, notamment en argent (Ag), en or (Au), en platine (Pt), en noir de platine, ou de dépôts de graphène dopés par des nanoparticules d'argent (Ag) ou d'or (Au), les nanoparticules étant fonctionnalisées par des liants du NO, notamment la guanylyl-cyclase ou des porphyrines.

Selon un mode de réalisation, la première partie comporte un corps fibreux pour amener le liquide biologique de la zone d'investigation à l'élément sensible grâce aux forces de capillarité.

Selon un mode de réalisation, la première partie comporte en outre un filtre configuré pour filtrer le liquide biologique à une entrée de l'élément sensible afin d'éviter de fausser la détection de NO par des éléments perturbateurs contenus dans le liquide biologique.

On peut prévoir que, dans l'appareil selon l'invention, la deuxième partie est disposée au-dessus de la première partie, la deuxième partie renfermant une électronique pour recueillir les mesures brutes du capteur électrochimique, pour les traduire en concentration de NO et assurer la transmission du signal avec éventuellement d'autres paramètres liés à l'environnement.

On peut prévoir que, dans l'appareil selon l'invention, l'appareil effectue et transmet des mesures à une fréquence fonction de l'état d'activité du sujet, cet état étant repéré au moyen d'un module gyroscopique et/ou accélérométrique de la deuxième partie de l'appareil.

Selon un mode de réalisation, l'appareil comprend un module de géolocalisation. Pour mieux faire comprendre l'objet de l'invention, on va en décrire ci-après, à titre d'exemple purement démonstratif et non limitatif, un mode de réalisation représenté sur le dessin annexé. Sur ce dessin :
- la figure 1 représente en perspective, une vue générale externe d'un appareil de détection selon l'invention ;
- la figure 2 représente une vue globale d'un sujet sur lequel a été mis en place un appareil selon l'invention ;
- la figure 3 représente une vue éclatée de l'appareil de la figure 1 ;
- la figure 4 représente un schéma-bloc correspondant au fonctionnement de l'appareil de la figure 3 ;
- la figure 5 représente une vue en plan d'un support planaire porteur de deux électrodes mises en place selon des courbes de Hilbert ;
- la figure 6 représente une courbe obtenue sur un sujet sain équipé d'un appareil selon l'invention, comme indiqué sur la figure 2 ;
- la figure 7 représente schématiquement un premier agencement du corps fibreux et de l'élément sensible de l'appareil ;
- la figure 8 représente schématiquement un deuxième agencement du corps fibreux et de l'élément sensible de l'appareil ;
- la figure 9 représente schématiquement un capteur électrochimique de l'élément sensible comprenant trois électrodes selon un premier mode de réalisation ;
- la figure 10 représente schématiquement un capteur électrochimique de l'élément sensible comprenant trois électrodes selon un deuxième mode de réalisation ;
- la figure 11 est une représentation schématique fonctionnelle d'un circuit microhydraulique agencé dans l'élément sensible ;
- la figure 12 est une vue en coupe de l'élément sensible selon un mode de réalisation.

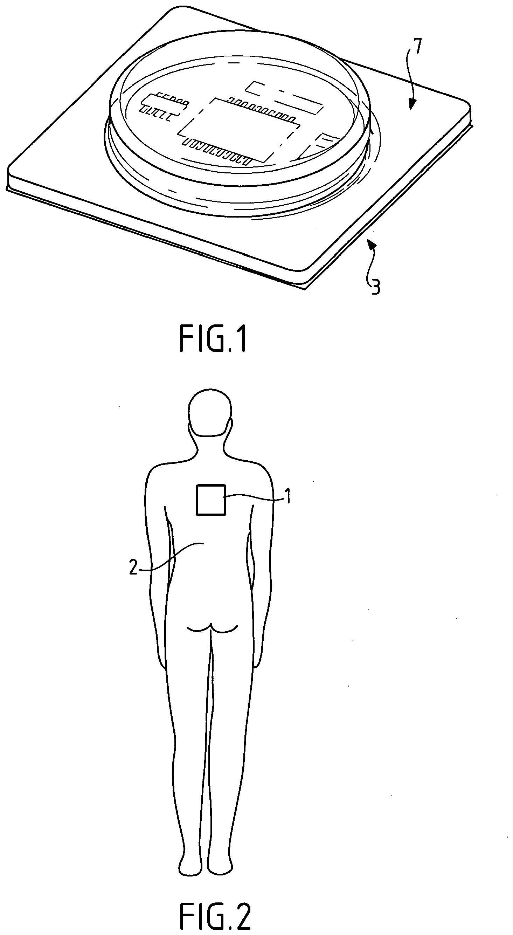

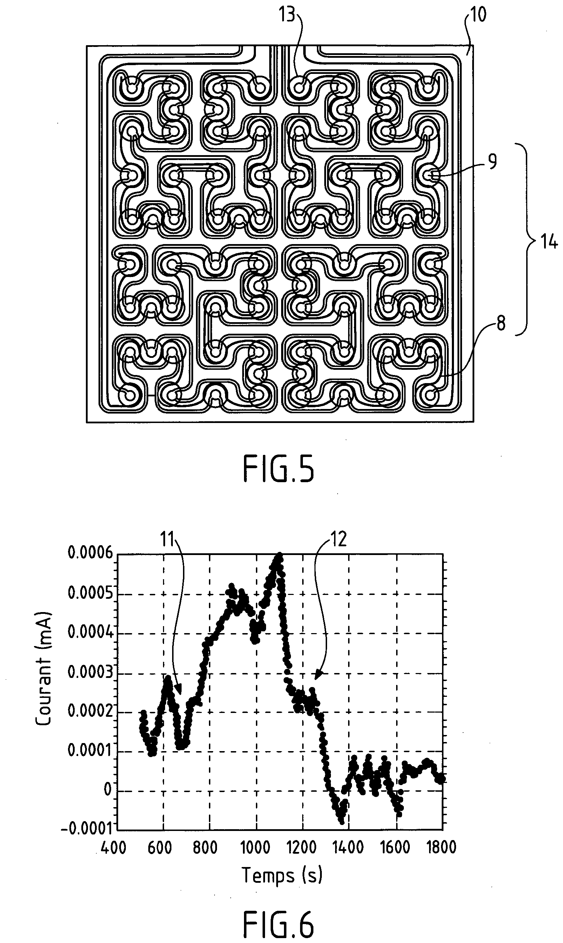

En se référant au dessin, on voit que l'appareil de détection selon l'invention est désigné par 1 dans son ensemble ; il est destiné à faire une mesure quantitative de NO sur un sujet humain sain. Dans l'exemple décrit, le sujet exerce une activité physique par l'utilisation d'un vélo correspondant à une puissance de 160 W. Comme le montre la figure 6, la détection de NO s'effectue dès le début du test (point 11) jusqu'à la fin du test (point 12), c'est-à-dire pendant une séquence de temps d'environ 500 secondes. En référence à la figure 1, l'appareil 1 se présente globalement sous la forme d'une pièce autocollante prenant dans l'exemple la forme d'un tampon autocollant, que l'on peut positionner directement sur la peau du sujet. Dans un mode de réalisation non illustré, la pièce autocollante est un pansement autocollant.

L'appareil selon l'invention comporte une embase de fixation 3 faite d'un matériau souple biocompatible et collant ; cette embase assure le maintien sur la peau de l'appareil complet ; la partie centrale 4a de l'embase 3 est un évidement circulaire où l'on positionne la première partie de l'appareil, qui permet de repérer la production de NO dans la zone d'investigation de la peau du sujet. L'évidement circulaire 4a permet donc le positionnement de la première partie de l'appareil de mesure directement sur la peau 2 du sujet. L'évidement 4a peut prendre une autre forme, par exemple choisie parmi l'ellipse, le triangle, le rectangle, le carré ou le polygone.

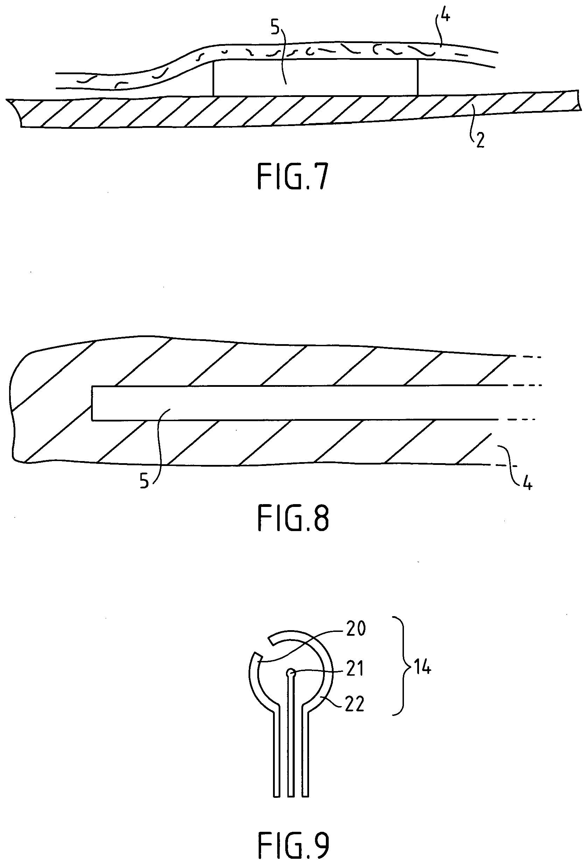

Cette première partie comprend un corps fibreux 4 qui est solidarisée d'un élément sensible 5 qu'il surmonte, comme illustré sur la figure 7, ou qu'il enveloppe, comme illustré sur la figure 8, pour constituer la base d'un empilement. Le corps fibreux remplit la fonction d'amener la sueur produite dans la zone d'investigation jusqu'à l'élément sensible 5 pour que le monoxyde d'azote dissous dans celle-ci soit détecté, puis d'évacuer la sueur une fois la mesure effectuée.

Un filtre 29 peut optionnellement être agencé entre le corps fibreux 4 et la ou les entrées de l'élément sensible 5. La fonction du filtre 29 est de filtrer la sueur pour éviter que certains éléments contenus naturellement dans celle-ci viennent perturber la mesure du NO dissous dans la sueur. Ces éléments perturbateurs sont par exemple le peroxynitrite (ONOO⁻) ou l'eau oxygénée (H₂O₂). L'élément sensible 5 assure la détection du NO au moyen d'un ou plusieurs capteurs électrochimiques 14, qui seront définis plus loin. Le capteur envoie ses informations sur un convertisseur 6a, qui alimente lui-même un processeur 6b, alimenté par un générateur d'énergie 6d associé audit élément sensible 5. Le processeur 6b alimente un système de communication-radio 6c, qui envoie l'information sur une instrumentation laquelle est susceptible de traduire cette information en une courbe telle que celle représentée sur la figure 6.

Sur cette figure 6, la partie constituant la mesure du NO produit pendant l'effort par le sujet, est celle qui est comprise entre les points 11 et 12 de la courbe. L'intégrale de la courbe de la figure 6 entre les points 11 et 12 correspond à un paramètre. Selon la valeur de ce paramètre, il est possible d'associer une pathologie comme l'artériosclérose II est aussi possible d'accompagner la gestion d'une fonction physiologique tel que le suivi de la biodisponibilité de l'alanine. En effet le précurseur naturel du NO dans l'organisme est un acide aminé appelé alanine. Le corps humain ne peut produire du NO en réponse à un effort que dans la limite de son stock d'alanine. Par voie de conséquence, l'appareil permet aussi de prédire le moment où le sujet ne sera plus en capacité de gérer sa vasodilatation, et donc le risque de se blesser.

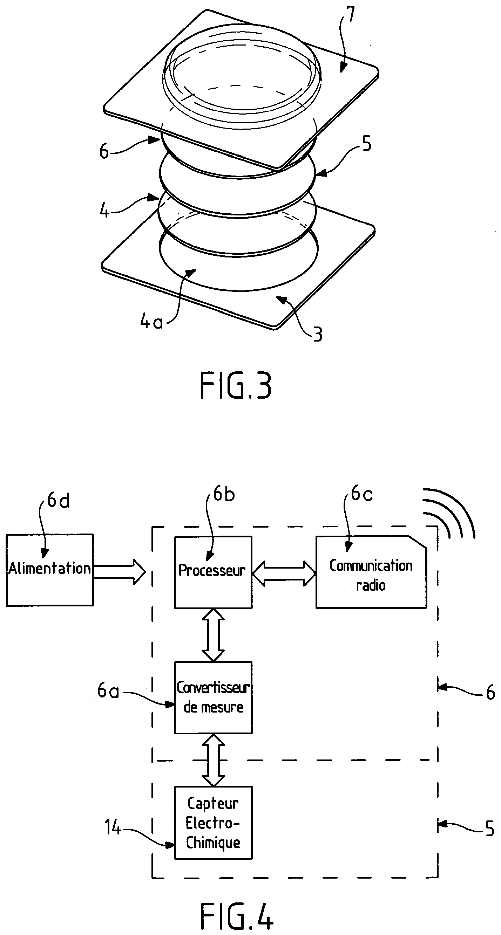

Tous les éléments assurant les différentes fonctions schématisées sur la figure 4, sont rassemblées dans une électronique embarquée, désignée par 6 dans son ensemble. Les constituants 4, 5 et 6 forment un empilement, qui est maintenu sur la peau du sujet au moyen d'une enveloppe souple et étanche à l'eau de type silicone, désignée par 7 dans son ensemble.

L'électronique embarquée de l'élément 6 assure les fonctions de contrôle des organes de l'élément sensible 5 ; elle comporte aussi une unité gyroscopique et accélérométrique pour connaitre l'orientation et les mouvements du sujet ainsi que le début et la fin de la séquence d'activité du sujet, et un capteur de température pour mesurer la température de la peau. Il est utile de connaitre la température de la peau pour pouvoir corréler la température et la dilatation des vaisseaux.

L'élément sensible de l'exemple décrit est électrochimique ; celui représenté sur la figure 5 comprend un capteur électrochimique 14 et un support planaire isolant 10 en polyimide. Le capteur électrochimique comprend deux électrodes 8 et 9 disposées sur une face du support planaire isolant 10 et entre lesquelles se trouve la sueur produite par le sujet dans la zone d'investigation, c'est-à-dire au droit de l'empilement 4, 5, 6. Dans l'élément sensible représenté sur la figure 5, on voit qu'il y a quatre unités identiques permettant chacune d'obtenir une mesure de NO. La mise en place de plusieurs unités sensibles permet avantageusement d'obtenir une cartographie cutanée de production de NO au sein de la zone recouverte.

En référence à la figure 7, le corps fibreux 4 et l'élément sensible 5 sont agencés d'une manière différente de la figure 3. Le support planaire isolant 10 est disposée directement sur la peau 2. La face du support 10 pourvue du capteur électrochimique est opposée à la face contre la peau 2. Le corps fibreux 4 présente une partie en contact avec la peau et une partie venant recouvrir la face du support comprenant le capteur électrochimique. En d'autres termes, le filtre 4 est à cheval sur la peau et le capteur électrochimique. Dans ce mode de réalisation, le corps fibreux comprend du coton ou un matériau non tissé.

En référence à la figure 8, un deuxième agencement du corps fibreux 4 et de l'élément sensible 5 est illustré. Le corps fibreux 4 vient prendre en sandwich l'élément sensible. Il en résulte qu'une partie du corps fibreux 4 est disposée contre la peau. Ensuite l'élément sensible 5 est disposé sur la partie corps fibreux contre la peau. La partie du corps fibreux 4 qui n'est pas disposée sur la peau est rabattue sur l'élément sensible 5 venant ainsi recouvrir le capteur.

Selon un premier mode de réalisation, le capteur électrochimique 14 comprend trois électrodes comme illustrées schématiquement sur la figure 9 : une électrode de référence 20, une électrode de travail 21 et une électrode auxiliaire 22. L'électrode de référence 20 est une électrode au chlorure d'argent (AgCI), l'électrode auxiliaire 22 est une électrode de platine (Pt) et l'électrode de travail 21 est une électrode à base de noir de platine. L'électrode de travail 21 présente la forme d'un disque. Ce disque est partiellement entouré par les électrodes de référence et auxiliaire, l'électrode de référence faisant face à l'électrode auxiliaire. Les dimensions du capteur électrochimique sont de l'ordre du millimètre.

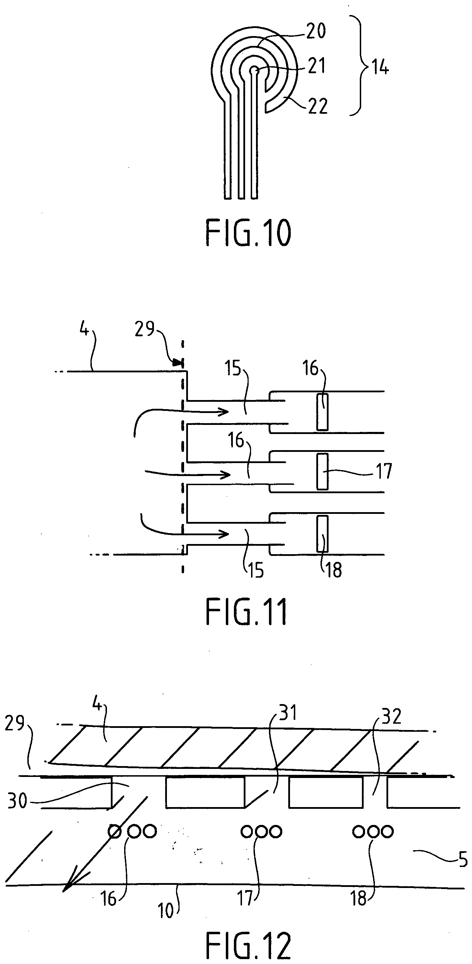

Selon un deuxième mode de réalisation illustré sur la figure 10, le capteur électronique comprend une électrode de référence 20, une électrode de travail 21 et une électrode auxiliaire 22. L'électrode de référence 20 est une électrode au chlorure d'argent (AgCI), l'électrode auxiliaire 22 est une électrode de platine (Pt) et l'électrode de travail 21 est une électrode à base de noir de platine. L'électrode de travail 21 présente la forme d'un disque. Ce disque est partiellement entouré par les électrodes de référence et auxiliaire. Les électrodes sont agencées de manière concentrique : l'électrode de travail 21 est partiellement entourée par l'électrode de référence 20, et l'électrode de référence 20 est elle-même entourée par l'électrode auxiliaire 22. Les dimensions du capteur électrochimique sont de l'ordre du millimètre.

Le capteur électrochimique de la figure 9 ou 10 peut être utilisé dans un circuit microhydraulique illustré schématiquement sur la figure 11.

Sur la figure 11, le corps fibreux 4 absorbe le liquide biologique, ici la sueur, et l'emmène vers trois microcanaux 15 tracés dans support planaire 10. Ces microcanaux 15 vont chacun amener la sueur vers des unités sensibles 16, 17, 18. Sur l'exemple représenté, il y a une unité sensible par microcanal 15. L'unité sensible 16 va détecter le monoxyde d'azote, l'unité sensible 17 va détecter le nitrite contenu dans la sueur, et l'unité sensible 18 va détecter l'eau oxygénée contenue dans la sueur. Le nitrite est principalement produit dans les cellules par la réaction entre l'oxygène superoxide (O2^{-•}) et le monoxyde d'azote. La détection de NO₂⁻ permet donc d'avoir une meilleure mesure de la concentration en NO.

Ainsi, chaque unité sensible est dédiée à la détection d'une espèce chimique. Chaque unité sensible est alimentée en courant ainsi chaque unité sensible est à un potentiel imposé pour effectuer une mesure stationnaire. L'unité sensible 18 est au potentiel redox de l'eau oxygénée (espèce oxydante) pour détecter l'eau oxygénée. Le traitement des données issues de l'unité sensible 18 donnera la quantité de H₂O₂. L'unité sensible 16 est au potentiel redox du NO (espèce oxydante) pour détecter le NO. Du fait que le potentiel redox de H₂O₂ est inférieur au potentiel redox de NO, l'unité sensible 16 détecte H₂O₂ en plus du NO. Le traitement des données issues de l'unité sensible 18 donnera la quantité de H₂O₂ et de NO pris ensemble. L'unité sensible 17 est au potentiel redox du nitrite (espèce oxydante) pour détecter le NO. Comme le potentiel redox de NO₂⁻ est supérieur au potentiel redox de H₂O₂ et NO, l'unité 17 détecte H₂O₂ et NO en plus de NO₂⁻. Le traitement des données issues de l'unité sensible 18 donnera la quantité de H₂O₂, de NO et de NO₂⁻ pris ensemble. Un autre traitement ultérieur des données produites par les unités sensibles 16, 17, 18 permet de déterminer par différence les quantités chaque espèces chimiques c'est-à-dire de NO, de H₂O₂ et de NO₂⁻.

Alternativement on peut utiliser une méthode par impulsion, chaque unité sensible sera alors capable de détecter chaque espèce. Après traitement des données, la quantité de chaque espèce présente pourra être déterminée.

En référence à la figure 12, l'élément sensible 5 comprend trois microcanaux 30, 31, 32 tracés dans l'épaisseur du support planaire isolant 10. Sur chaque paroi de fond des microcanaux 30, 31, 32 sont placées les unités sensibles 16, 17, et 18. L'unité sensible 16 est configurée pour détecter le monoxyde d'azote, l'unité sensible 17 est configurée pour détecter le nitrite contenu dans la sueur, et l'unité sensible 18 est configurée pour détecter l'eau oxygénée contenue dans la sueur. Chaque unité sensible 16, 17, 18 comprend trois capteurs 14. Au-dessus du support planaire isolant est placé un filtre 29. Le filtre vient recouvrir les microcanaux. Enfin un corps fibreux 4 est placé sur le filtre 29.

Le corps fibreux 4 absorbe le liquide biologique, ici la sueur, et l'emmène vers les trois microcanaux 30, 31, 32 grâce aux forces de capillarité. Lorsque la sueur drainée par le corps fibreux 4 arrive niveau des microcanaux, la sueur est filtrée par le filtre 29 pour enlever certains éléments perturbateurs, puis elle est transportée par les microcanaux 30, 31, 32 au moins jusqu'aux unités sensibles 16, 17, 18. Les capteurs de l'unité sensible 16 détecte alors le NO, les capteurs de l'unité sensible 17 détecte le nitrite et les capteurs de l'unité sensible 18 détecte l'eau oxygénée.

Dans un mode de réalisation non représenté, lorsque l'élément sensible comprend plusieurs unités sensibles dont au moins une est dédiée à la détection d'une espèce chimique autre que le NO, par exemple l'eau oxygénée, alors le filtre 29 peut être supprimé.

Les intensités de courant que l'on obtient avec l'appareil selon l'invention, sont comprises entre le pico et le milliampère.

Bien que l'invention ait été décrite en liaison avec plusieurs modes de réalisation particuliers, il est bien évident qu'elle n'y est nullement limitée et qu'elle comprend tous les équivalents techniques des moyens décrits ainsi que leurs combinaisons si celles-ci entrent dans le cadre de l'invention.

L'usage du verbe « comporter », « comprendre » ou « inclure » et de ses formes conjuguées n'exclut pas la présence d'autres éléments ou d'autres étapes que ceux énoncés dans une revendication.

Dans les revendications, tout signe de référence entre parenthèses ne saurait être interprété comme une limitation de la revendication.

## Revendications

1. Procédé pour détecter, sur un sujet, une quantité de NO produite par ledit sujet au cours d'une séquence d'un état d'activité prédéfini, **caractérisé en ce qu'**on choisit une zone d'investigation d'un épiderme (2) dudit sujet, on y repère, de façon directe et continue, la production de NO dissous dans un liquide biologique provenant de l'épiderme au moyen d'un appareil formé d'une première partie (4), portée par ladite zone d'investigation et maintenue sur elle de façon étanche, cette première partie (4) étant solidarisée d'un élément sensible (5), qui assure la détection du NO au moyen d'un capteur électrochimique (14), et que l'on envoie par ledit capteur électrochimique, grâce à un générateur d'énergie associé audit élément sensible (5), un signal, dont la lecture permet la détection souhaitée.

2. Procédé selon la revendication 1, **caractérisé en ce qu'**on met en oeuvre au moins un capteur électrochimique, qui fournit le signal comme résultat d'une mesure électrochimique faite en utilisant le liquide biologique, produite par le sujet dans la zone d'investigation, comme électrolyte entre deux électrodes de travail (8,9) portées par un support planaire isolant (10).

3. Procédé selon la revendication 2, **caractérisé en ce qu'**on associe aux deux électrodes de travail (8,9) une électrode de référence.

4. Procédé selon la revendication 2 ou 3, **caractérisé en ce que** le capteur électrochimique comporte une pluralité de capteurs électrochimiques, dont les signaux sont associés pour améliorer le signal de sortie.

5. Procédé selon l'une des revendications 2 à 4, **caractérisé en ce que** le tracé des électrodes (8,9) par rapport à leur support suit une courbe de Hilbert pour améliorer la puissance du signal de sortie par unité de surface du support (10).

6. Procédé selon l'une des revendications 2 à 5, **caractérisé en ce qu'**on effectue les mesures au droit d'orifices (13) prévus dans le support planaire, qui se trouve au droit des tracés conducteurs des électrodes (8,9).

7. Procédé selon l'une des revendications 2 à 6, **caractérisé en ce que** les électrodes sont constituées de dépôts métalliques, notamment en argent (Ag), en or (Au), en platine (Pt), en noir de platine, ou de dépôts de graphène dopés par des nanoparticules d'argent (Ag) ou d'or (Au), les nanoparticules étant fonctionnalisées par des liants du NO, notamment la guanylyl-cyclase ou des porphyrines.

8. Procédé selon l'une des revendications 1 à 7, **caractérisé en ce que** l'appareil comporte en outre une deuxième partie (6) disposée au-dessus de la première partie (4), la deuxième partie (6) renfermant une électronique pour recueillir les mesures brutes du capteur électrochimique, les traduire en concentration de NO et assurer la transmission du signal avec éventuellement d'autres paramètres liés à l'environnement.

9. Procédé selon l'une des revendications 1 à 8, **caractérisé en ce que** l'appareil effectue et transmet des mesures à une fréquence fonction de l'état d'activité du sujet, cet état étant repéré au moyen d'un module gyroscopique et/ou accélérométrique de la deuxième partie (5) de l'appareil.

10. Appareil (1) de détection pour détecter, sur un sujet, une quantité de NO produite par ledit sujet au cours d'une séquence d'un état d'activité prédéfini, ledit appareil comportant une première partie (4) destinée à être portée par une zone d'investigation d'un épiderme (2) dudit sujet et maintenue sur elle de façon étanche pour repérer de façon directe et continue, la production de NO dans un liquide biologique provenant de l'épiderme, la première partie étant solidarisée d'un élément sensible (5), qui assure la détection de NO au moyen d'un capteur électrochimique (14), et une deuxième partie (6) configurée pour envoyer, grâce à un générateur d'énergie (5d) associé audit élément sensible (5), un signal, dont la lecture permet la détection souhaitée.

11. Appareil selon la revendication 10, **caractérisé en ce que** l'élément sensible fournit le signal comme résultat d'une mesure électrochimique faite en utilisant le liquide biologique, produit par le sujet dans la zone d'investigation, comme électrolyte entre deux électrodes de travail (8,9) portées par un support planaire isolant (10).

12. Appareil selon la revendication 11, **caractérisé en ce qu'**une électrode de référence est associée aux deux électrodes de travail.

13. Appareil selon l'une des revendications 11 à 12, **caractérisé en ce que** le support planaire isolant (10) comprend au moins un microcanal (15, 30, 31, 33) de sorte à guider le liquide biologique vers le capteur électrochimique (14).

14. Appareil selon l'une des revendications 10 ou 13, **caractérisé en ce que** l'élément sensible comporte une pluralité de capteurs électrochimiques semblables, dont les signaux sont associés pour améliorer le signal de sortie.

15. Appareil selon l'une des revendications 11 à 14, **caractérisé en ce que** le tracé des électrodes de travail (8,9) par rapport à leur support (10) suit une courbe de Hilbert pour améliorer la force du signal de sortie par unité de surface du support.

16. Appareil selon l'une des revendications 11 à 15, **caractérisé en ce que** les mesures sont effectuées au droit d'orifices (13) prévus dans le support planaire (10), qui se trouve au droit des tracés conducteurs des électrodes (8,9).

17. Appareil selon l'une des revendications 11 à 16, **caractérisé en ce que** les électrodes de travail (8,9) sont constituées de dépôts métalliques, notamment en argent (Ag), en or (Au), en platine (Pt), en noir de platine, ou de dépôts de graphène dopées par des nanoparticules d'argent (Ag) ou d'or (Au), les nanoparticules étant fonctionnalisées par des liants du NO, notamment la guanylyl-cyclase ou des porphyrines.

18. Appareil selon l'une des revendications 10 ou 17, **caractérisé en ce que** l'élément sensible comporte une pluralité de capteurs électrochimiques (14) répartis en une pluralité d'unités sensibles (16, 17, 18), et **en ce que** chaque unité sensible est configurée pour détecter au moins une espèce chimique.

19. Appareil selon les revendications 13 et 18 prises en combinaison, **caractérisé en ce que** le support planaire isolant comprend une pluralité de microcanaux (30, 31, 32), et **caractérisé en ce que** chaque canal comprend une unité sensible.

20. Appareil selon l'une des revendications 10 à 19, **caractérisé en ce que** la première partie comporte un corps fibreux pour amener le liquide biologique de la zone d'investigation à l'élément sensible grâce aux forces de capillarité.

21. Appareil selon la revendication 20, **caractérisé en ce que** la première partie comporte en outre un filtre (29) configuré pour filtrer le liquide biologique à une entrée de l'élément sensible afin d'éviter de fausser la détection de NO par des éléments perturbateurs contenus dans le liquide biologique

22. Appareil selon l'une des revendications 10 à 21, **caractérisé en ce que** la deuxième partie est disposée au-dessus de la première partie (4), la deuxième partie renfermant une électronique pour recueillir les mesures brutes du capteur électrochimique, pour les traduire en concentration de NO et assurer la transmission du signal avec éventuellement d'autres paramètres liés à l'environnement.

23. Appareil selon l'une des revendications 10 à 22, **caractérisé en ce que** l'appareil comporte un module gyroscopique et/ou accélérométrique pour détecter l'état d'activité du sujet et que l'appareil est configuré pour effectuer et transmettre des mesures à une fréquence fonction de l'état d'activité du sujet.

## Patentansprüche

1. Verfahren zum Erfassen einer Menge an NO, die von einem Subjekt während einer Sequenz eines vordefinierten Aktivitätszustands produziert wird, **dadurch gekennzeichnet, dass** ein Untersuchungsgebiet der Epidermis (2) des Subjekts ausgewählt wird, und dass die Produktion von NO, das in einer von der Epidermis stammenden biologischen Flüssigkeit gelöst ist, dort direkt und kontinuierlich mittels einer Vorrichtung erfasst wird, die aus einem ersten Teil (4) besteht, der vom Untersuchungsgebiet getragen und daran dicht gehalten wird, wobei der erste Teil (4) fest mit einem Sensorbauteil (5) verbunden ist, das den Nachweis von NO mittels eines elektrochemischen Sensors (14) sichert, und das von diesem elektrochemischen Sensor durch Nutzung einer mit dem Sensorbauteil (5) verbundenen Energiequelle ein Signal gesendet wird, dessen Ablesen die gewünschte Erfassung ermöglicht.

2. Verfahren nach Anspruch 1, **dadurch gekennzeichnet, dass** mindestens ein elektrochemischer Sensor verwendet wird, der das Signal als Ergebnis einer elektrochemischen Messung liefert, die unter Verwendung der von dem Subjekt im Untersuchungsgebiet produzierten biologischen Flüssigkeit als Elektrolyt zwischen zwei Arbeitselektroden (8, 9) durchgeführt wird, die von einem isolierenden planaren Träger (10) getragen werden.

3. Verfahren nach Anspruch 2, **dadurch gekennzeichnet, dass** den zwei Arbeitselektroden (8, 9) eine Referenzelektrode zugeordnet ist.

4. Verfahren nach Anspruch 2 oder 3, **dadurch gekennzeichnet, dass** der elektrochemische Sensor eine Mehrzahl von elektrochemischen Sensoren umfasst, deren Signale miteinander verknüpft werden, um das Ausgangssignal zu verbessern.

5. Verfahren nach einem der Ansprüche 2 bis 4, **dadurch gekennzeichnet, dass** der Verlauf der Elektroden (8, 9) relativ zu ihrem Träger einer Hilbert-Kurve folgt, um die Leistung des Ausgangssignals pro Flächeneinheit des Trägers (10) zu verbessern.

6. Verfahren nach einem der Ansprüche 2 bis 5, **dadurch gekennzeichnet, dass** die Messungen direkt unter Öffnungen (13) im planaren Träger durchgeführt werden, die sich direkt unter den Leiterbahnen der Elektroden (8, 9) befinden.

7. Verfahren nach einem der Ansprüche 2 bis 6, **dadurch gekennzeichnet, dass** die Elektroden aus Metallüberzügen bestehen, insbesondere aus Silber (Ag), Gold (Au), Platin (Pt), Platinschwarz, oder aus mit Silber- (Ag) oder Gold- (Au) Nanopartikeln dotierten Graphenüberzügen bestehen, wobei die Nanopartikel mit NO-Bindemitteln, insbesondere Guanylylcyclase oder Porphyrinen, funktionalisiert sind.

8. Verfahren nach einem der Ansprüche 1 bis 7, **dadurch gekennzeichnet, dass** die Vorrichtung außerdem einen zweiten Teil (6) umfasst, der über dem ersten Teil (4) angeordnet ist, wobei der zweite Teil (6) eine Elektronik enthält, um die Rohmessungen des elektrochemischen Sensors zu sammeln, sie in NO-Konzentration zu übersetzen und die Übertragung des Signals, eventuell zusammen mit anderen umgebungsbezogenen Parametern, zu sichern.

9. Verfahren nach einem der Ansprüche 1 bis 8, **dadurch gekennzeichnet, dass** die Vorrichtung Messungen mit einer vom Aktivitätszustand des Subjekts abhängigen Frequenz durchführt und überträgt, wobei dieser Zustand mittels eines gyroskopischen und/oder akzelerometrischen Moduls des zweiten Teils (5) der Vorrichtung festgestellt wird.

10. Erfassungsvorrichtung (1) zum Erfassen einer Menge an NO, die von einem Subjekt während einer Sequenz eines vordefinierten Aktivitätszustands produziert wird, wobei die Vorrichtung einen ersten Teil (4) umfasst, der dazu bestimmt ist, von einem Untersuchungsgebiet der Epidermis (2) des Subjekts getragen und dicht daran gehalten zu werden, um die Produktion von NO in einer aus der Epidermis stammenden biologischen Flüssigkeit direkt und kontinuierlich zu erfassen, wobei der erste Teil fest mit einem Sensorbauteil (5) verbunden ist, das den Nachweis von NO mittels eines elektrochemischen Sensors (14) sichert, und einen zweiten Teil (6), der so eingerichtet ist, dass er durch Nutzung einer mit dem Sensorbauteil (5) verbundenen Energiequelle (5d) ein Signalsenden kann, dessen Ablesen die gewünschte Erfassung ermöglicht.

11. Vorrichtung nach Anspruch 10, **dadurch gekennzeichnet, dass** das Sensorbauteil das Signal als Ergebnis einer elektrochemischen Messung liefert, die unter Verwendung der von dem Subjekt im Untersuchungsgebiet produzierten biologischen Flüssigkeit als Elektrolyt zwischen zwei Arbeitselektroden (8, 9) durchgeführt wird, die von einem isolierenden planaren Träger (10) getragen werden.

12. Vorrichtung nach Anspruch 11, **dadurch gekennzeichnet, dass** den zwei Arbeitselektroden (8, 9) eine Referenzelektrode zugeordnet ist.

13. Vorrichtung nach einem der Ansprüche 11 bis 12, **dadurch gekennzeichnet, dass** der isolierende planare Träger (10) mindestens einen Mikrokanal (15, 30, 31, 33) umfasst, um die biologische Flüssigkeit zum elektrochemischen Sensor (14) zu leiten.

14. Vorrichtung nach einem der Ansprüche 10 oder 13, **dadurch gekennzeichnet, dass** das Sensorbauteil eine Vielzahl ähnlicher elektrochemischer Sensoren umfasst, deren Signale zur Verbesserung des Ausgangssignals verknüpft werden.

15. Vorrichtung nach einem der Ansprüche 11 bis 14, **dadurch gekennzeichnet, dass** der Verlauf der Arbeitselektroden (8, 9) relativ zu ihrem Träger (10) einer Hilbert-Kurve folgt, um die Stärke des Ausgangssignals pro Flächeneinheit des Trägers zu verbessern.

16. Vorrichtung nach einem der Ansprüche 11 bis 15, **dadurch gekennzeichnet, dass** die Messungen direkt unter Öffnungen (13) im planaren Träger durchgeführt werden, die sich direkt unter den Leiterbahnen der Elektroden (8, 9) befinden.

17. Vorrichtung nach einem der Ansprüche 11 bis 16, **dadurch gekennzeichnet, dass** die Arbeitselektroden aus Metallüberzügen bestehen, insbesondere aus Silber (Ag), Gold (Au), Platin (Pt), Platinschwarz, oder aus mit Silber- (Ag) oder Gold- (Au) Nanopartikeln dotierten Graphenüberzügen bestehen, wobei die Nanopartikel mit NO-Bindemitteln, insbesondere Guanylylcyclase oder Porphyrinen, funktionalisiert sind.

18. Vorrichtung nach einem der Ansprüche 10 oder 17, **dadurch gekennzeichnet, dass** das Sensorbauteil eine Mehrzahl von elektrochemischen Sensoren (14) umfasst, die in eine Vielzahl von Sensoreinheiten (16, 17, 18) aufgeteilt sind, und dass jede Sensoreinheit so eingerichtet ist, dass sie mindestens eine chemische Spezies erfasst.

19. Vorrichtung nach den Ansprüchen 13 und 18 in Kombination, **dadurch gekennzeichnet, dass** der isolierende planare Träger eine Vielzahl von Mikrokanälen (30, 31, 32) umfasst, und dass jeder Kanal eine Sensoreinheit umfasst.

20. Vorrichtung nach einem der Ansprüche 10 bis 19, **dadurch gekennzeichnet, dass** der erste Teil einen faserigen Körper umfasst, um die biologische Flüssigkeit aus dem Untersuchungsgebiet durch Kapillarkräfte zum Sensorbauteil zu leiten.

21. Vorrichtung nach Anspruch 20, **dadurch gekennzeichnet, dass** der erste Teil zusätzlich einen Filter (29) umfasst, der so eingerichtet ist, dass er die biologische Flüssigkeit an einem Eingang des Sensorbauteils zu filtern, um eine Verfälschung der NO-Erfassung durch in der biologischen Flüssigkeit enthaltene Störelemente zu vermeiden.

22. Vorrichtung nach einem der Ansprüche 10 bis 21, **dadurch gekennzeichnet, dass** der zweite Teil über dem ersten Teil (4) angeordnet ist, wobei der zweite Teil (6) eine Elektronik enthält, um die Rohmessungen des elektrochemischen Sensors zu sammeln, sie in NO-Konzentration zu übersetzen und die Übertragung des Signals, eventuell zusammen mit anderen umgebungsbezogenen Parametern, zu sichern.

23. Vorrichtung nach einem der Ansprüche 10 bis 22, **dadurch gekennzeichnet, dass** die Vorrichtung ein gyroskopisches und/oder akzelerometrisches Modul zum Erfassen des Aktivitätszustands des Subjekts enthält und dass die Vorrichtung so eingerichtet ist, dass sie Messungen mit einer Frequenz durchführt und überträgt, die vom Aktivitätszustand des Subjekts abhängt.

## Claims

1. A process for detecting, in a subject, an amount of NO produced by said subject in the course of a sequence of a predefined activity state, **characterized in that** an investigation zone of an epidermis (2) of said subject is chosen, the production of NO dissolved in a biological liquid originating from the epidermis is tracked therein, directly and continuously, by means of a device formed of a first part (4), borne by said investigation zone and held thereon in a leaktight manner, this first part (4) being attached to a sensing element (5), which carries out the detection of the NO by means of an electrochemical sensor (14), and that, owing to an energy generator associated with said sensing element (5), a signal is sent by said electrochemical sensor, the reading of which signal enables the desired detection.

2. The process as claimed in claim 1, **characterized in that** use is made of at least one electrochemical sensor, which provides the signal as a result of an electrochemical measurement taken using the biological liquid, produced by the subject in the investigation zone, as electrolyte between two work electrodes (8,9) borne by an insulating planar support (10).

3. The process as claimed in claim 2, **characterized in that** a reference electrode is connected to the two work electrodes (8,9).

4. The process as claimed in claim 2 or 3, **characterized in that** the electrochemical sensor comprises a plurality of electrochemical sensors, the signals of which are combined to improve the output signal.

5. The process as claimed in one of claims 2 to 4, **characterized in that** the pattern of the electrodes (8,9) relative to their support follows a Hilbert curve in order to improve the power of the output signal per unit area of the support (10).

6. The process as claimed in one of claims 2 to 5, **characterized in that** the measurements are carried out in line with orifices (13) provided in the planar support, which is in line with the conductive patterns of the electrodes (8,9).

7. The process as claimed in one of claims 2 to 6, **characterized in that** the electrodes consist of metal deposits, in particular deposits of silver (Ag), gold (Au), platinum (Pt), and platinum black, or graphene deposits doped by nanoparticles of silver (Ag) or of gold (Au), the nanoparticles being functionalized by binders of NO, in particular guanylyl-cyclase or porphyrins.

8. The process as claimed in one of claims 1 to 7, **characterized in that** the device further comprises a second part (6) positioned above the first part (4), the second part (6) containing electronics for receiving the raw measurements from the electrochemical sensor, converting them into an NO concentration and ensuring the transmission of the signal possibly with other parameters linked to the environment.

9. The process as claimed in one of claims 1 to 8, **characterized in that** the device carries out and transmits measurements at a frequency that is a function of the activity state of the subject, this state being tracked by means of a gyroscopic and/or accelerometric module of the second part (5) of the device.

10. A detection device (1) for detecting, in a subject, an amount of NO produced by said subject in the course of a sequence of a predefined activity state, said device comprising a first part (4) intended to be borne by an investigation zone of an epidermis (2) of said subject and held thereon in a leaktight manner in order to track, directly and continuously, the production of NO in a biological liquid originating from the epidermis, the first part being attached to a sensing element (5), which carries out the detection of NO by means of an electrochemical sensor (14), and a second part (6) configured to send, owing to an energy generator (5d) associated with said sensing element (5), a signal, the reading of which enables the desired detection.

11. The device as claimed in claim 10, **characterized in that** the sensing element provides the signal as a result of an electrochemical measurement taken using the biological liquid, produced by the subject in the investigation zone, as electrolyte between two work electrodes (8,9) borne by an insulating planar support (10).

12. The device as claimed in claim 11, **characterized in that** a reference electrode is connected to the two work electrodes.

13. The device as claimed in either of claims 11 and 12, **characterized in that** the insulating planar support (10) comprises at least one microchannel (15, 30, 31, 33) so as to guide the biological liquid to the electrochemical sensor (14).

14. The device as claimed in either of claims 10 and 13, **characterized in that** the sensing element comprises a plurality of similar electrochemical sensors, the signals of which are combined to improve the output signal.

15. The device as claimed in one of claims 11 to 14, **characterized in that** the pattern of the work electrodes (8,9) relative to their support (10) follows a Hilbert curve in order to improve the strength of the output signal per unit area of the support.

16. The device as claimed in one of claims 11 to 15, **characterized in that** the measurements are carried out in line with orifices (13) provided in the planar support (10), which is in line with the conductive patterns of the electrodes (8,9).

17. The device as claimed in one of claims 11 to 16, **characterized in that** the work electrodes (8,9) consist of metal deposits, in particular deposits of silver (Ag), gold (Au), platinum (Pt), and platinum black, or graphene deposits doped by nanoparticles of silver (Ag) or of gold (Au), the nanoparticles being functionalized by binders of NO, in particular guanylyl-cyclase or porphyrins.

18. The device as claimed in either of claims 10 and 17, **characterized in that** the sensing element comprises a plurality of electrochemical sensors (14) distributed in a plurality of sensing units (16, 17, 18), and **in that** each sensing unit is configured to detect at least one chemical species.

19. The device as claimed in claims 13 and 18 taken in combination, **characterized in that** the insulating planar support comprises a plurality of microchannels (30, 31, 32), and **characterized in that** each channel comprises a sensing unit.

20. The device as claimed in one of claims 10 to 19, **characterized in that** the first part comprises a fibrous body in order to convey the biological liquid from the investigation zone to the sensing element by means of capillary forces.

21. The device as claimed in claim 20, **characterized in that** the first part further comprises a filter (29) configured to filter the biological liquid at an inlet of the sensing element in order to avoid distorting the detection of NO by interfering elements contained in the biological liquid.

22. The device as claimed in one of claims 10 to 21, **characterized in that** the second part is positioned above the first part (4), the second part containing electronics for receiving the raw measurements from the electrochemical sensor, for converting them into an NO concentration and ensuring the transmission of the signal possibly with other parameters linked to the environment.

23. The device as claimed in one of claims 10 to 22, **characterized in that** the device comprises a gyroscopic and/or accelerometric module to detect the activity state of the subject and that the device is configured to carry out and transmit measurements at a frequency that is a function of the activity state of the subject.
